# EUROPEAN PATENT APPLICATION

(11) **EP 4 791 119 A1**
(43) Date of publication of application: **12.08.2026**
(21) Application number: 25155907.6
(22) Date of filing: 05.02.2025
(51) Int. Cl.: H05B 47/11, A61N 5/06, H05B 47/105, G01J 3/02, H05B 45/22, A61M 21/00

(54) **CONTROL SYSTEM FOR CONTROLLING LIGHT EMISSION OF A LUMINAIRE**

(71) Applicant: Tridonic GmbH & Co. KG, 6851 Dornbirn (AT)
(72) Inventor: Meitz, Simon Michael, 6851 Dornbirn (AT); Bakk, Istvan, 6851 Dornbirn (AT)
(74) Representative: Rupp, Christian

(57) **Abstract**

The present invention provides a control system (10) for controlling light emission of a luminaire (20) towards a surface (30). The control system (10) comprises a sensor (1) configured to detect light and having a sensitivity in the melanopic region, and a control unit (2). The sensor (1) is configured to be arranged such that the sensor (1) detects light (50) reflected from the surface (30) being illuminated by the luminaire when the luminaire emits light (40). The control unit (2) is configured to determine a melanopic equivalent daylight illuminance, MEDI, of the light emission (40) of the luminaire (20) using a detection result of the sensor (1). The control unit (2) is configured to control the light emission (40) of the luminaire (20) according to the determined MEDI.

## Description

The present invention relates to a control system for controlling light emission of a luminaire, and to a luminaire comprising such a control system.

Light emission of a luminaire may be controlled according to light conditions present in the area, in which the luminaire is installed. For example, a light sensor may measure the amount of sunlight in the area so that the light emission of the luminaire can be adapted to the measured amount of sunlight.

Lighting may be provided directly or indirectly. When the light emission is provided indirectly, a luminaire e.g. being installed in a room, may emit light towards a ceiling where it is reflected in order to provide an indirect lighting or indirect illumination by the luminaire. The light emission by the luminaire may be referred to as artificial lighting. Controlling light emission of a luminaire allows setting the amount and type of light being present in an area, such as a room, in which the luminaire is installed. The amount of light may be set according to a lighting condition desired by a person being present in the area. For example, in case the person wants to work in said area the desired lighting is usually different compared to a case when the person wants to relax in the area.

Like sunlight, artificial lighting of a luminaire has an influence on the circadian rhythm of a person being present in the area being illuminated by the artificial light. Especially, the way in which light enters the human eye and, thus, reaches the ganglion cells in the retina determines the extent to which the light influences the circadian rhythm of a person.

It is an object of the present invention to provide means that allow controlling light emission of a luminaire such that the light emission influences the circadian rhythm of a person.

These and other objects, which become apparent upon reading the following description, are solved by the subject-matter of the independent claim. The dependent claims refer to preferred embodiments of the invention.

According to a first aspect of the invention, a control system for controlling light emission of a luminaire towards a surface is provided, the luminaire being configured to emit light with wavelengths in the melanopic region. The control system comprises a sensor configured to detect light and having a sensitivity in the melanopic region. The control system comprises a control unit. The sensor is configured to be arranged such that the sensor detects light reflected from the surface being illuminated by the luminaire when the luminaire emits light. The control unit is configured to determine a melanopic equivalent daylight illuminance (MEDI) of the light emission of the luminaire using a detection result of the sensor. The control unit is configured to control the light emission of the luminaire according to the determined MEDI.

In other words, the first aspect of the present invention allows determining how artificial lighting (i.e. the light emission of a luminaire) being reflected at a surface influences the circadian rhythm of a person and, thus, determining how indirect lighting provided by a luminaire influences the circadian rhythm of the person. Namely, a light source, such as the sun or a luminaire (being an artificial light source), has an impact on the visual perception of a person as well as on the circadian rhythm. In the human eye the ganglion cells of the retina (also referred to as intrinsically photosensitive retinal ganglion cells, ipRGCs) are not involved in the visual perception (i.e. forming an image of the environment perceived by the eye), but they are involved in the circadian rhythm as they synchronize the internal clock with the 24 h daily cycle based on the incoming melanopic light. The ganglion cells comprise a photopigment called melanopsin that is sensitive to blue light. In case of light having a greater degree of blue light and a greater intensity the ganglion cells cause the hormone melatonin having a calming effect on the human body to be repressed and the hormone cortisol having an activating effect (awaking effect) on the human body to increase. Accordingly, light having a lower degree of blue light supports release of melatonin which has a calming effect and, thus, helps a person to get tired and fall asleep. The effect of light on the melanopsin of the ganglion cells of the human eye and, thus, on the hormones melatonin and cortisol is called melanopic effect. That is, light comprising wavelength in the melanopic region has an effect on the ganglion cells and, thus, on the circadian rhythm of a person. The melanopic region is in the wavelength range between 460 and 500 nm and, thus, encompasses blue light.

Moreover, the angle of incidence of light entering the human eye has an influence as this determines to what extent the light falls onto the ganglion cells on the retina. Light having an angle of incident with regard to the horizon between 0° and 45°, i.e. from the upper half space, may have the greatest effect on the circadian rhythm. Namely, the ganglion cells are arranged in the lower half of the retina so that light reaching the human eye from the upper half space is most effective for influencing the circadian rhythm. Thus, indirect lighting from the ceiling (i.e. reflected from the ceiling) may especially influence the circadian rhythm. For example, the melanopic activation by light may come from the direction of the ceiling (i.e. from above) for a sitting observer. The melanopic equivalent daylight illumination (MEDI) of a light emission of a light source, such as a luminaire, is a value that describes how much the light emission affects the ganglion cells and, thus, the circadian rhythm.

Since the sensor is sensitive in the melanopic region it allows measuring the part of the light emission that has an effect on the ganglion cells and, thus, influences the circadian rhythm. Moreover, since the sensor is configured to be arranged such that it detects the light reflected from the surface, the sensor may measure the part of the indirect light emission of the luminaire that influences the circadian rhythm. Thus, the detection result of the sensor allows determining the MEDI of the indirect light emission of the luminaire. This allows controlling the light emission of the luminaire towards the surface and, thus, an indirect light emission of an area, in which the surface is present, by the luminaire according to the determined MEDI and, thus, by considering the effect of the light emission of the luminaire on the circadian rhythm of a person being present in the area. This allows controlling light emission of the luminaire such that the light emission influences the circadian rhythm of the person.

For example, the surface may be a ceiling of a room and the luminaire may be configured to provide illumination by emitting light towards the ceiling when being installed in the room. This provides an indirect lighting via the ceiling of the room. Such a luminaire may be for example a free-standing luminaire (FSL) or a hanging luminaire installed at the ceiling, wherein the luminaire is configured to emit light in the direction of the ceiling when being positioned on the ground (in case of the free-standing luminaire) or installed at the ceiling (in the case of the hanging luminaire). The term "pendant luminaire" may be used as a synonym for the term "hanging luminaire".

The luminaire may comprise one or more light emitting diodes (LEDs) as a light source for providing the light emission. The light emission of the luminaire towards the surface may be referred as "indirect light emission". Namely, when the luminaire is installed in a room, where the surface (such as a ceiling or surface of a desk) is present, the light emission towards the surface cause light to be reflected at the surface. The reflected light then provides illumination to the room. That is, the luminaire provides an indirect light emission via the surface to the area (e.g. room) in which the surface is present.

The sensor may be referred to as light sensor. The sensor having a sensitivity in the melanopic region means that the sensor is configured to measure light that has wavelengths in the melanopic region and, thus, to measure light that has an effect on the ganglion cells of the human eyes (i.e. an effect on the circadian rhythm).

The control unit may be or comprise at least one of processor(s), microprocessor(s), controller(s), microcontroller(s), application specific integrated circuit(s) (ASIC(s)), field programmable gate array(s) (FPGA(s)), etc.

The sensor being configured to be arranged such that the sensor detects light reflected from the surface being illuminated by the luminaire means that the sensor is configured to be arranged such that the sensor detects an indirect light emission of the luminaire via the surface when the luminaire emits light towards the surface. Emitting light towards the surface results in an illumination of the surface by the luminaire. The sensor being configured to be arranged such that the sensor detects light reflected from the surface being illuminated by the luminaire means that the sensor is configured to be arranged such that a detection side of the sensor, via which light may enter the sensor in order to be measured (i.e. detected), is directed towards the surface. This allows the light being reflected at the surface to travel to the detection side of the sensor and, thus, to be detected by the sensor.

The sensor may be configured to be arranged at a light emission side of the luminaire such that the sensor detects the light reflected from the surface being illuminated by the luminaire when the luminaire emits light. The light emission side of the luminaire is a side of the luminaire directed towards the surface via which the light emission of the luminaire is provided. That is, the light emission of the luminaire towards (i.e. in the direction) of the surface is a light emission by the light emissions side of the luminaire.

Determining the MEDI of the light emission of the luminaire, i.e. the light emission of the luminaire towards the surface, means determining to what extent the light emission affects the circadian rhythm and, thus, the indirect light emission via the surface affects the circadian rhythm of a person.

The control unit may be configured to control the light emission of the luminaire according to the determined MEDI by providing control signals to a light engine and/or a driver circuit of the luminaire that drives (i.e. electrically supplies) the light source of the luminaire, the light source being configured to emit light towards the surface and, thus, providing the light emission of the luminaire.

The control unit may be configured to control the luminaire to emit light with a spectral power distribution of a correlated color temperature (CCT) of 6500 Kelvin and a melanopic daylight efficacy ratio (MDER) equal to or greater than 1.15 in case the determined MEDI is greater than 0 lx and smaller than or equal to 250 lx. The control unit may be configured to control the luminaire to emit light with a spectral power distribution of a CCT of 4000 Kelvin and a MDER equal to or greater than 0.9 and smaller than 1.15 in case the determined MEDI is greater than 250 lx and smaller than or equal to 500 lx. The control unit may be configured to control the luminaire to emit light with a spectral power distribution of a CCT of 4000 Kelvin and a MDER equal to or greater than 0.6 and smaller than 0.9 in case the determined MEDI is greater than 500 lx and smaller than or equal to 750 lx.

Herein, "lx" stands for "lux", which is the unit of illuminance, which represents the amount of luminous flux per unit area. The melanopic daylight efficacy ratio (MDER), in short "melanopic ratio", of light being emitted by the luminaire allows computing the melanopic light intensity. The term "melanopic lux" may be used as a synonym for the term "melanopic light intensity". The greater the melanopic light intensity of light the greater the influence of the light on the ganglion cells and, thus, circadian rhythm and vice versa. The photopic light intensity of light represents the amount of light having an effect on the visual perception of the light by the human eye. The terms "photopic lux" and "photopic illuminance" may be used as synonyms for the term "photopic light intensity". The photopic light intensity is the photometric equivalent of irradiance. That is, the greater the photopic light intensity of a light emission the greater the brightness of the light emission perceived by the human eye and vice versa. The sunlight, i.e. daylight, regulates the circadian rhythm of a person and is the reference for the MDER. The reference is called CIE D65 standard illuminant being the International Commission on Illumination's (CIE) standard illuminant. It represents white light with a CCT of 6500 Kelvin. The MDER of the light emission of a light source, such as the light emission of the luminaire, represents a comparison between the spectral power distribution (SPD) of the light emission of the light source and the SPD of the CIE D65 standard illuminant.

The melanopic light intensity of a light emission of a luminaire may be computed by multiplying the photopic light intensity of the luminaire by the MDER of the light emission of the luminaire. Thus, when the luminaire emits light with a SPD having a lower MDER the photopic light intensity of the light emission is to be higher in order to achieve a desired melanopic light intensity and vice versa. The amount (i.e. the photopic light intensity) and the spectral composition (i.e. SPD of the light) of the light emitted by the luminaire affects the amount of melanopic lux, i.e. the melanopic light intensity, of the light emitted by the luminaire. The MDER of a luminaire and, thus, its light emission may vary with the CCT of the light emission assuming a constant photopic light intensity.

For example, for a good reflecting, white ceiling the control unit may control the luminaire so that the light is dimmed down (less energy consumption and thus sustainability). For example, for a slightly yellowish ceiling (blue absorbent) the control unit may control the luminaire so that light with a SPD having a greater MDER is emitted by the luminaire.

Optionally, the control unit is configured to control the luminaire to emit no light in case the determined MEDI is greater than 750 lx.

Optionally, the sensor comprises a single sensor channel having a spectral sensitivity with a peak between 460 nm and 500 nm. Optionally the single sensor channel has a spectral sensitivity with a peak between 475 nm and 485 nm.

That is, the spectral sensitivity may have a peak in a wavelength range between 460 nm and 500 nm, optionally between 475 nm and 485 nm.

Optionally, the control unit is configured to compute the MEDI by multiplying the detection result of the sensor with a factor.

The detection result of the sensor may represent a relative value of the photopic illuminance, wherein the factor may be the unit "melanopic lx", which may be also referred to as "MEDI lx". In the aforementioned case, the detection result of the sensor may be defined as unitless (e.g. arbitrary units). Thus, the MEDI is a value with the unit "lx", representing the melanopic light intensity.

Optionally, the sensor comprises multiple sensor channels having different spectral sensitivities, wherein a sensor channel of the multiple sensor channels has a spectral sensitivity with a peak between 460 nm and 500 nm. Optionally, the sensor channel of the multiple sensor channels has a spectral sensitivity with a peak between 475 nm and 485 nm.

That is, the spectral sensitivity of one sensor channel of the multiple sensor channels may have a peak in a wavelength range between 460 nm and 500 nm, optionally between 475 nm and 485 nm. The different spectral sensitivities of the multiple may have a respective peak in the wavelength range between 380 nm and 750 nm. The different spectral sensitivities of the multiple may have a respective peak in a wavelength range perceivable by the human eye. The different sensor channels may have different spectral sensitivities such that a spectrum of sunlight may be detected by the sensor. The sensor comprising multiple sensor channels may be referred to as "multi-spectral sensor". The sensor may comprise one or more filters for one or more of the multiple sensor channels, which only transmit color-corresponding wavelengths. That is, the one or more sensor channels may be one or more color sensor channels. The sensor may have multiple areas of the same filter distributed over the total area of the detection side of the sensor (e.g. two of the same placed diagonally apart). The sensor may have as a sensor channel a white color channel (may be referred to as clear channel) that is configured to sense a wide range of wavelengths. For example the white color channel may be or may function as a standard silicon light sensor.

Optionally, the control unit is configured to compute the MEDI by summing up a multiplication of a detection result of each sensor channel of the multiple sensor channels with a respective factor.

The detection result of each sensor channel may represent a relative value of the irradiance in the sensor channel's spectral range, wherein the factor may be the unit "melanopic lx", which may be also referred to as "MEDI lx". In the aforementioned case, the detection result of a respective sensor channel may be defined as unitless (e.g. arbitrary units).

Optionally, the control system comprises a Lambertian diffusor that is arranged such that the sensor is configured to receive light only via the Lambertian diffusor.

The term "Lambertian diffusing element" may be used as a synonym for the term "Lambertian diffusor". The Lambertian diffusor allows filtering light travelling towards the sensor such that light reflected at the surface is allowed to enter the sensor, whereas an amount of incoming sunlight from an environment of the control system is insignificant. Thus, the diffusor allows the sensor to measure the light reflected at the surface without sunlight that may be present in an environment of the surface, when the sensor is arranged in the environment and the detection side of the sensor is directed towards the surface. This allows determining a total reflectivity of the surface using the detection result of the sensor.

Optionally, the control system comprises an optical dome structure that is arranged such that the sensor is configured to receive light only via the optical dome structure.

The optical dome structure allows reflected light and sunlight to reach the sensor. This allows the sensor to detect the indirect light emission of the luminaire via the surface as well as sunlight being present. That is, this allows the sensor to detect a total light in the environment of the surface, the total light comprising the artificial light emitted by the luminaire and reflected at the surface and the sunlight, when sunlight is present in the environment.

Optionally, an infrared channel of the multiple sensor channels is configured to detect infrared radiation. The control unit may be configured to determine sunlight detected by the sensor using a detection result of the infrared channel of the multiple sensor channels and ignore the determined sunlight for computing the MEDI.

Optionally, the control unit is configured to determine a photopic illuminance of light entering the sensor using the detection result of the sensor. The control unit may be configured to control the light emission of the luminaire such that the greater the determined photopic illuminance of the light entering the sensor the more the light emission of the luminaire is dimmed and vice versa.

This allows adjusting the photopic illuminance of the light. As described above, this allows changing the melanopic light intensity without changing the MDER of the light emission and, thus, allows changing the effect of the light emission on the circadian rhythm. Such dimming allows harvesting natural light (sunlight) and increasing the efficiency through dimming / switching the luminaire off.

Optionally, the control unit is configured to determine a spectral reflectivity of the surface by computing a quotient of light detected by the sensor and light emitted by the luminaire.

The spectral reflectivity of the surface may represent the ratio of radiation reflected by the surface to the radiation impacting or incoming at the surface (e.g. as a result of the light emission by the luminaire towards the surface) as a function of the wavelength. The term "spectral reflectance curve" may be used as a synonym for the term "spectral reflectivity". Thus, the spectral reflectivity may comprise for each wavelength of the wavelengths of the radiation (e.g. the light emission of the luminaire) a respective percentage value. For example, the control system may be configured to determine the spectral reflectivity of the surface with regard to the melanopic region.

Optionally, the control system is configured to determine a total reflectivity of the surface by computing a quotient of light detected by the sensor and light emitted by the luminaire.

The total reflectivity of the surface may represent the ratio of the photopic illuminance of light reflected at the surface to the photopic illuminance of light impacting or incoming at the surface (e.g. as a result of the light emission by the luminaire towards the surface). The total reflectivity is a single percentage value. Optionally, the control unit is configured to determine that the luminaire and the surface is suited for an indirect illumination via the surface in case the determined total reflectivity is greater than 80%.

For determining the spectral reflectivity and/or total reflectivity of the surface using the detection result of the sensor the distance between the sensor and the surface and/or the distance between the luminaire and the surface may be considered. When the sensor is installed at the luminaire, the distance between the luminaire and the surface and the distance between the sensor and the surface is the same. For example, in case the surface is the ceiling of a room and the sensor is installed at a height below the ceiling such that the detection side of the sensor is directed towards the ceiling, then either the room height may be considered when known by the control system or a height difference between the ceiling and the sensor and/or luminaire may be considered. For the room height a default value (e.g. 2.7 m) may be stored in the control unit, which may be changed according to the actual height. For determining the spectral reflectivity and/or total reflectivity of the surface the dimming level of the light emission may be set to a set dimming level (e.g. 100%) and a lighting mode with a set spectral power distribution (SPD) of the light emission of the luminaire towards the surface may be set.

Optionally, the control system is a sensor device, the sensor and control unit being arranged in a housing of the sensor device. Optionally, the control system comprises a sensor device, the sensor being arranged in a housing of the sensor device.

The control system allows adjusting lighting parameters, such as photopic illuminance, CCT, etc., of the light emission of the luminaire by determining the MEDI of the light emission and controlling the light emission according to the determined MEDI. This allows adjusting the aforementioned lighting parameters without directly measuring them.

The control unit and the sensor may be arranged inside a Zhaga book 20 housing. The control unit may be configured to control the luminaire over different known lighting standards, such as DALI-1, DALI-2, Casambi etc.

Herein, the description with regard to the indirect light emission is correspondingly valid for a direct light emission of the luminaire, where such direct light emission is directed to an object having a surface, such as the surface of a desk, at which the light is reflected.

In order to achieve the control system according to the first aspect of the present invention, some or all of the above described optional features may be combined with each other.

According to a second aspect of the invention, a luminaire for emitting light towards a surface is provided. The luminaire comprises the control system according to the first aspect of the invention, as described above. The luminaire is configured to emit light with wavelengths in the melanopic region. The sensor of the control system is arranged at the luminaire such that the sensor detects light reflected from the surface being illuminated by the luminaire when the luminaire emits light.

The luminaire may comprise a tunable white light source for the light emission. That is, the tunable white light source may emit or contribute to the emission of light with wavelengths in the melanopic region. The luminaire may comprise one or more light emitting diodes (LEDs) as the light source for providing the light emission. Since the luminaire is configured to emit light towards the surface, the luminaire is configured for an indirect light emission (via the surface). The luminaire is configured to emit light with different spectral power distributions (SPDs). That is, the luminaire is configured to change the SPD of its light emission.

Optionally, the luminaire is configured to emit light towards a surface of a room, such as a ceiling or wall, when the luminaire is installed in the room. Optionally, the luminaire is configured to emit light towards a surface of an object, such as a desk, being present in the room when the luminaire is installed in the room for illuminating the object.

The luminaire may be configured to emit light with one of a spectral power distribution of a CCT of 6500 Kelvin and a MDER equal to or greater than 1.15, a spectral power distribution of a CCT of 4000 Kelvin and a MDER equal to or greater than 0.9 and smaller than 1.15; and a spectral power distribution of a CCT of 4000 Kelvin and a MDER equal to or greater than 0.6 and smaller than 0.9. The luminaire may emit light with different CCTs, for example with a CCT in a range between 6500 Kelvin and 3000 Kelvin. The luminaire may emit light with different MDER, for example with a MDER greater than 0.6, 0.9 or 1.15.

Optionally, the control unit of the control system is a control unit of the luminaire configured to control operation of the luminaire.

The above description with regard to the control system according to the first aspect of the present invention is also valid for the luminaire according to the second aspect of the present invention. The above description with regard to the luminaire according to the second aspect of the present invention is also valid for the control system according to the first aspect of the present invention.

The luminaire according to the second aspect of the invention achieves the same advantages as the control system according to the first aspect of the invention.

All steps which are performed by the various entities described in the present application as well as the functionalities described to be performed by the various entities are intended to mean that the respective entity is adapted to or configured to perform the respective steps and functionalities.

In the following, the invention is described exemplarily with reference to the enclosed figures (FIGs.), in which
- **FIG. 1**: shows an example of a control system according to the present invention,
- **FIG. 2**: shows two examples of implementation forms of the control system of FIG. 1,
- **FIG. 3**: shows an example of a luminaire according to the present invention,
- **FIG. 4**: shows an example of a luminaire according to the present invention,
- **FIG. 5**: shows an example of an implementation form of the control system of FIG. 1,
- **FIG. 6**: shows an example of an implementation form of the control system of FIG. 1,
- **FIG. 7**: shows an example of different spectral power distributions of light emittable by a luminaire according to the invention,
- **FIG. 8**: shows an example of a sensitivity of an implementation form of the sensor of the control system of Figure 1, and
- **FIG. 9**: shows an example of a sensitivity of an implementation form of the sensor of the control system of Figure 1.

In the FIGs. (FIGs.), corresponding elements have the same reference signs. The size of elements in the FIGs. is not to scale and may be different compared to a real life implementation in order to highlight details of the embodiments.

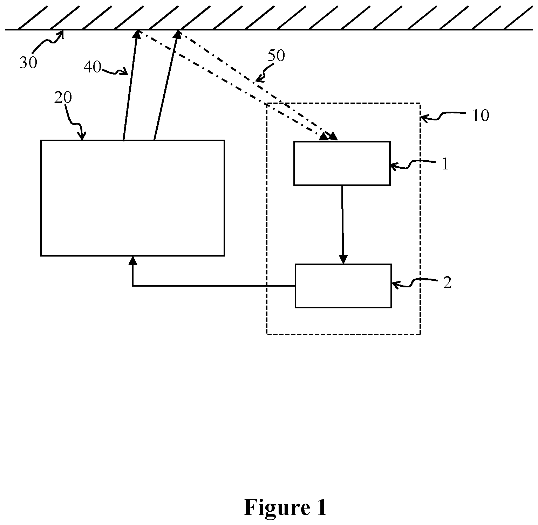

**FIG. 1** shows an example of a control system according to the present invention. The control system of FIG. 1 is an example of the control system according to the first aspect of the invention. The description of the control system of the first aspect is correspondingly valid for the control system of FIG. 1.

The control system 10 of FIG. 1 is a control system for controlling light emission of a luminaire 20 towards a surface 30. The surface may be a ceiling of a room so that the luminaire 20 is configured to provide an indirect light emission in the room by emitting light towards the ceiling. Optionally, the surface may be the surface of an object, such as a desk, being illuminated by the light emission of the luminaire 20 (not shown in FIG. 1). The luminaire 20 is configured to emit light with wavelengths in the melanopic region, i.e. light having an influence on the ganglion cells of the human eye and, thus, on the circadian rhythm of a person. The description of the luminaire according to the second aspect is correspondingly valid for the luminaire 20.

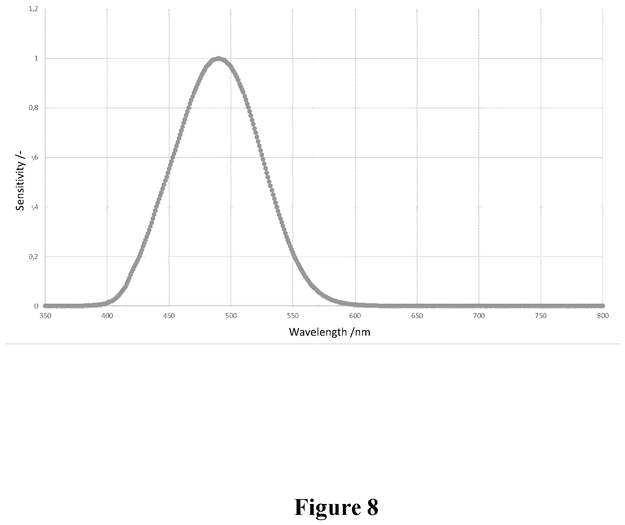

The control system 10 comprises a sensor 1 configured to detect light and having a sensitivity in the melanopic region. The sensor 1 may comprise a single sensor channel having a spectral sensitivity with a peak between 460 nm and 500 nm, optionally 475 nm and 485 nm. **FIG. 8** shows an example of the sensitivity of such an implementation form of the sensor 1. The vertical axis of the graph of FIG. 8 shows the sensitivity of the sensor 1 and the horizontal axis of the graph of FIG. 8 shows the wavelength in nm. As can be derived from FIG. 8, the single sensor channel has a sensitivity in the melanopic region and, thus, can measure light that has an effect of the circadian rhythm of a person by influencing the ganglion cells of the human eye. The curve of FIG. 8 represents the melanopic sensitivity curve.

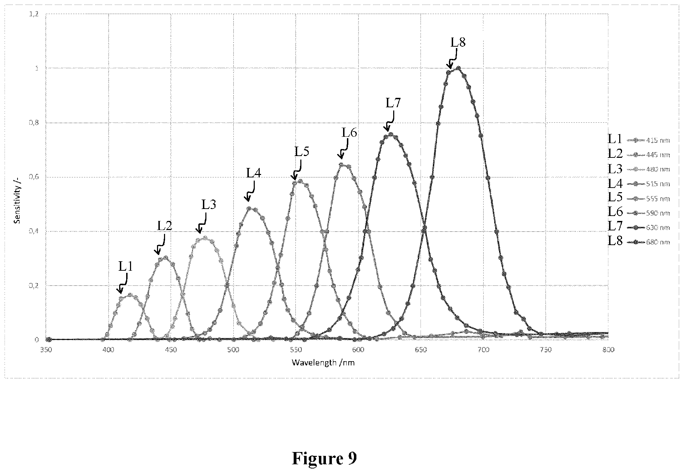

Alternatively, the sensor 1 may comprise multiple sensor channels having different spectral sensitivities, wherein a sensor channel of the multiple sensor channels has a spectral sensitivity with a peak between 460 nm and 500 nm, optionally 475 nm and 485 nm. **FIG. 9** shows an example of the sensitivity of such an implementation form of the sensor 1. The vertical axis of the graph of FIG. 9 shows the sensitivity relative to 680 nm of the sensor 1 and the horizontal axis of the graph of FIG. 9 shows the wavelength in nm. According to the example of FIG. 9, the sensor 1 has eight sensor channels. Each curve L1 to L8 represents the spectral sensitivity of a respective sensor channel of the multiple sensor channels. The number of sensor channels indicated by FIG. 9 is only by way of example and may be different.

As shown in FIG. 1, the control system 10 comprises a control unit 2. The control unit 2 is configured to receive detection results from the sensor 1 (as indicated by the arrow from the box representing the sensor 1 to the box representing the control unit 2 in FIG. 1). The control unit 2 is configured to control the luminaire 20 and, thus, the light emission of the luminaire 20 (as indicated by the arrow from the box representing the control unit 2 to the box representing the luminaire 20 in FIG. 1). For example, the control unit 2 is configured to provide control signals to the luminaire, e.g. a driver for driving (i.e. electrically supplying) the light source (e.g. one or more LEDs) of the luminaire and/or a control unit of the luminaire 20 in order to control the light emission of the luminaire 20. Herein, communication between components, such as the sensor 1 and control unit 2 or the control unit 2 and the luminaire 20 may be performed according to any method known in the art. That is, wireless communication (such as Bluetooth communication, wireless local area network (WLAN) communication, etc.) and/or wired communication (such as via a wired data bus) may be used.

As shown in FIG. 1, the sensor 1 is configured to be arranged such that the sensor 1 detects light 50 reflected from the surface 30 being illuminated by the luminaire 20 when the luminaire 20 emits light 40 towards the surface 30. In the FIGs. light emitted by the luminaire 20 is represented by solid arrows, wherein light emitted towards the surface 30 is labeled by the reference sign "40", and light reflected by/from the surface 30 is represented by dashed single dotted arrows and labeled with the reference sign "50". In other words, the sensor 1 is configured to be arranged in an environment of the surface 30 and, thus, in an environment of the luminaire 20 emitting light towards the surface 30 such that the detection side of the sensor 1 is directed towards the surface 30 allowing the light 50 reflected from the surface 30 to be detected/measured by the sensor 1 when the luminaire 20 emits light towards the surface 30 (i.e. illuminates the surface 30).

Optionally, the sensor 1 may be arranged at a different location compared to the location of the luminaire. For example, the sensor 1 may be arranged at a second luminaire (not shown in FIG. 1) that is arranged together with the luminaire 20 in the environment of the surface 30, e.g. a room in which the surface 30 is present. The surface may be for example a ceiling of the room. Optionally, the sensor 1 may be arranged at a light emission side of the second luminaire, the light emission side being directed towards the surface 30 when the second luminaire is installed for emitting light towards the surface 30 and, thus, illuminating the surface 30 for a providing an indirect light emission (via the surface 30). The control system 10 may be part of a lighting system comprising multiple luminaires, wherein the control unit 2 may control the light emission of at least one luminaire of the multiple luminaires as outlined herein. Optionally, the control unit 2 may be a central control unit of such a lighting system. The control system 10 may be used e.g. in a (wireless) network of luminaires, where the control unit 2 may be configured to determine a contribution of all luminaires on the lighting level at a certain point in time.

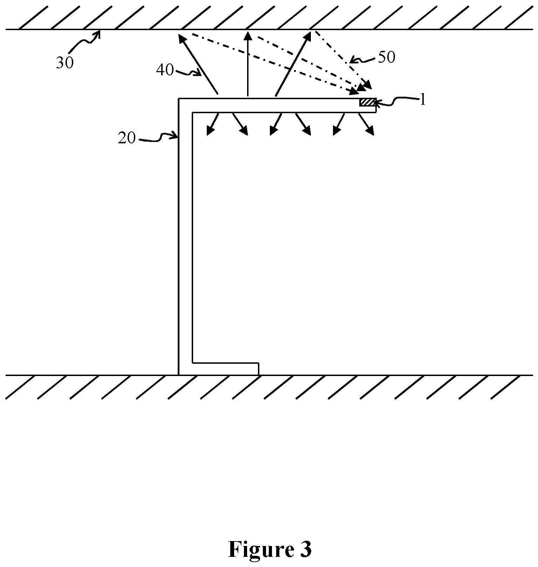

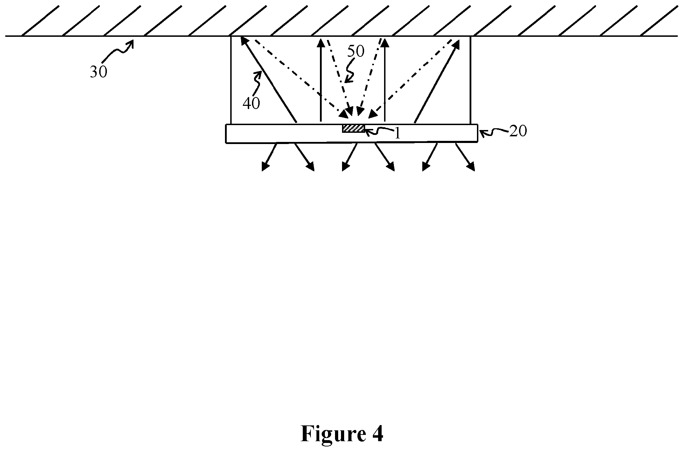

Optionally, the sensor 1 may be arranged at the luminaire 20 such that the detection side of the sensor 1 is directed towards the surface 30 so that the sensor 1 detects/measures the light 50 reflected by/from the surface 30 when the surface 30 is illuminated by the luminaire 20. For example, the sensor 1 may be arranged at a light emission side of the luminaire 20, the light emission side being directed towards the surface 30 when the luminaire 20 is installed for emitting light towards the surface 30 and, thus, illuminating the surface 30 for a providing an indirect light emission (via the surface 30). **FIGs. 3** **and** **4** show two examples of such an arrangement of the sensor 1 at the luminaire 20 for two different types of luminaire 20. In FIGs. 3 and 4 the control unit 2 is not shown.

As shown in FIG. 3, the luminaire 20 may be a free-standing luminaire, wherein a top side of the head part of the free-standing luminaire 20 represent a light emission side of the luminaire 20 for emitting light towards the surface 30. As shown in FIG. 3, the sensor 1 may be arranged at the light emission side of the luminaire and, thus, at the head part of the free-standing luminaire 20. The position of the sensor 1 at the end/tip of the head part of the luminaire 20 shown in FIG. 3 is only by way of example and, thus, the sensor 1 may be located differently at the head part of the luminaire 2. Optionally, the sensor 1 may be arranged differently at the free-standing luminaire 20 as long as the detection side of the sensor 1 is directed towards the surface 30 allowing the reflected light 40 (reflected at the surface 30) to be detected/measured by the sensor 1. For example, the sensor may be arranged at the pole part of the free-standing luminaire 20 that holds the head part above ground. As shown in FIG. 3, the free-standing luminaire may optionally also emit light in the direction of the ground and, thus, provide a direct light emission.

As shown in FIG. 4, the luminaire 20 may be a hanging luminaire and the surface 30 may be the ceiling of a room at which the hanging luminaire 20 may be installed. A top side of the hanging luminaire 20 being installed at the ceiling represent a light emission side of the luminaire 20 for emitting light towards the ceiling 30. As shown in FIG. 3, the sensor 1 may be arranged at the light emission side of the luminaire and, thus, at the top side of the hanging luminaire 20. The position of the sensor 1 in the center of the light emission side of the luminaire 20 shown in FIG. 4 is only by way of example and, thus, the sensor 1 may be located differently at the light emission side of the luminaire 20. The arrangement at the center is a placement that allows the ratio between the artificial light and sunlight to be highest. Optionally, the sensor 1 may be arranged differently at the hanging luminaire 20 as long as the detection side of the sensor 1 is directed towards the surface 30 allowing the reflected light 50 (reflected at the surface 30) to be detected/measured by the sensor 1. As shown in FIG. 4, the hanging luminaire may optionally also emit light in the direction of the ground and, thus, provide a direct light emission.

The control unit 2 of the control system 10 of FIG. 1 is configured to determine a melanopic equivalent daylight illuminance (MEDI) of the light emission of the luminaire 20 using a detection result of the sensor 1.

For example, in case the sensor 1 comprises the single sensor channel having a spectral sensitivity with a peak between 460 nm and 500 nm, optionally 475 nm and 485 nm, the control unit 2 may be configured to compute the MEDI by multiplying the detection result of the sensor 1 with a factor. This may be represented by the following equation, in which *E_{raw}* represents the raw sensor value provided as a detection result by the sensor 1 and f represents a factor that can be set with a simple calibration: $MEDI = f ∗ {E}_{raw}$

For example, in case the sensor 1 comprises the multiple sensor channels having different spectral sensitivities, wherein a sensor channel of the multiple sensor channels has a spectral sensitivity with a peak between 460 nm and 500 n, optionally 475 nm and 485 nm, the control unit 2 may be configured to compute the MEDI by summing up a multiplication of a detection result of each sensor channel of the multiple sensor channels with a respective factor. This may be represented by the following equation, in which Eᵢ represents the raw sensor value as a detection result of a respective sensor channel of the sensor 1 and fᵢ represents a respective factor for each sensor channel that can be set with a simple calibration, wherein n is the number of sensor channels: $MEDI = {f}_{1} ∗ {E}_{1} + {f}_{2} ∗ {E}_{2} + ⋯ + {f}_{n} ∗ {E}_{n} = {\sum }_{i = 1}^{n} {f}_{i} ∗ {E}_{i}$

The control unit 2 may be configured to determine the photopic illuminance of the light entering the sensor by directly using the raw sensor value E_{raw} of the sensor or the sum of the raw sensor values of the different sensor channels. Optionally, for determining the photopic illuminance of the light entering the sensor the control unit 2 may be configured to use the raw sensor value E_{raw} of the sensor or the sum of the raw sensor values of the different sensor channels and as outlined above for the MEDI a factor (in case of using the raw sensor value E_{raw}) or factors (in case of using the raw sensor values of the different sensor channels). The aforementioned factor or factors for determining the photopic illuminance is/are different to the factor or factors for determining the MEDI. Optionally, the control unit 2 may determine the photopic illuminance of the light entering the sensor 1 by using the raw sensor value of a clear channel (white color channel) of the multiple sensor channels when the sensor 1 has the multiple sensor channels.

The control unit 2 may be configured to determine the tristimulus (X, Y, Z) weighted irradiance of the light entering the sensor 1 using the detection result of the sensor 1. The control unit 2 may be configured to directly determine the CCT of the light entering the sensor 1 using the detection result of the sensor 1. The control unit may be configured to determine the CCT of the light entering the sensor 1 using the tristimulus (X, Y, Z) weighted irradiance of the light. For example this may be done using the following formula: $CCT \left(x, y\right) = - 499 {n}^{3} + 325 {n}^{2} - 6823.3 n + 5520.33 ,$ wherein $n = \frac{x - {x}_{e}}{y - {y}_{e}} ; {x}_{e} = 0.3320 ; {y}_{e} = 0.1858 ; x = \frac{X}{X + Y + Z} ; and y = \frac{Y}{X + Y + Z}$

The control unit 2 may be configured to determine the color rendering factors Rᵢ and with that the color rendering index (CRI) of the light entering the sensor 1 using the detection result of the sensor 1. In case the sensor 1 comprises the multiple sensor channels, the control unit may determine the spectral properties, such as the tristimulus (X, Y, Z) weighted irradiance, CCT, Rᵢ, CRI etc., of the light entering the sensor 1 by using a certain weighting of the detection results of the different sensor channels of the sensor 1.

The control unit 2 is configured to control the light emission of the luminaire according to the determined MEDI. The control unit 2 may be configured to control the luminaire 20 to emit light with a spectral power distribution of CCT of 6500 Kelvin and a MDER equal to or greater than 1.15 in case the determined MEDI is greater than 0 lx and smaller than or equal to 250 lx. The control unit 2 may be configured to control the luminaire 20 to emit light with a spectral power distribution of a CCT of 4000 Kelvin and a MDER equal to or greater than 0.9 and smaller than 1.15 in case the determined MEDI is greater than 250 lx and smaller than or equal to 500 lx. The control unit 2 may be configured to control the luminaire 20 to emit light with a spectral power distribution of a CCT of 4000 Kelvin and a MDER equal to or greater than 0.6 and smaller than 0.9 in case the determined MEDI is greater than 500 lx and smaller than or equal to 750 lx. The control unit 2 may be configured to control the luminaire 20 to emit no light in case the determined MEDI is greater than 750 lx.

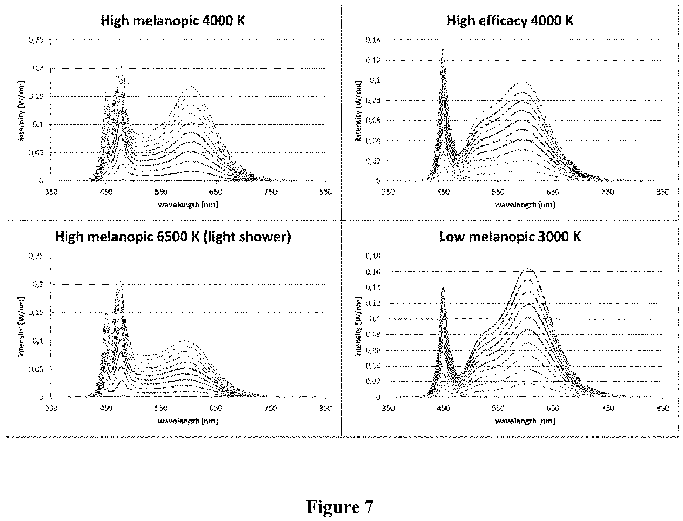

**FIG. 7** shows examples of such different spectral power distributions of light emittable by the luminaire. The vertical axis of each graph of FIG. 7 represents intensity in W/nm and the horizontal axis represents the wavelength in nm. The top left graph of FIG. 7 labelled "high melanopic 4000 K" shows an example of a spectral power distribution of a CCT of 4000 Kelvin and a MDER equal to or greater than 0.9 and smaller than 1.15. The top right graph of FIG. 7 labelled "high efficacy 4000 K" shows an example of a spectral power distribution of a CCT of 4000 Kelvin and a MDER equal to or greater than 0.6 and smaller than 0.9. The bottom left graph of FIG. 7 labelled "high melanopic 6500 K (light shower)" shows an example of a spectral power distribution of a CCT of 6500 Kelvin and a MDER equal to or greater than 1.15. The bottom right graph of FIG. 7 labelled "low melanopic 3000 K" shows an example of a spectral power distribution of a CCT of 3000 Kelvin and a MDER smaller than the MDER of the other distributions shown in FIG. 7 and, thus, with the lowest melanopic influence on the human eye.

The control unit 2 may be configured to control the light emission of the luminaire 20 such that the greater the determined photopic illuminance of the light entering the sensor 1 the more the light emission of the luminaire 20 is dimmed and vice versa. The photopic illuminance can be used for dimming the luminaire 20, so that a constant light level / lx is reached. For example in case the total reflectivity of the surface (at which the light is reflected for the indirect light emission) is low, the control unit may set a higher dimming level and vice versa. The dimming level may be between 0% and 100%, wherein a dimming level of 0% means no light emission and a dimming level of 100% means maximum light emission. The more the luminaire is dimmed the lower the dimming level of the light emission of the luminaire and vice versa. The control unit 2 may use the determined photopic illuminance in combination with e.g. the MEDI to adjust an optimal spectrum with the right dimming level of the light emission.

Optionally, an infrared channel of the multiple sensor channels of the sensor 1 is configured to detect infrared radiation. The control unit 2 may be configured to determine sunlight detected by the sensor 1 using a detection result of the infrared channel of the multiple sensor channels and ignore the determined sunlight for computing the MEDI. The information on the determined sunlight may be used to control shades that may be present in the area where the luminaire 20 and the control system 10 are installed.

The control unit 2 may use the determined CCT of the light entering the sensor 1 for controlling the light emission by the luminaire 20 such that a defined lighting scene with a certain light color is reached. If e.g. the measured CCT is < 4000 K and the desired value is 4000 K, the control unit 2 may control the luminaire by setting a lighting mode with a higher CCT (cf. FIG. 7). The CRI is a measure of the quality of light. Thus, the control unit 2 may use the determined CRI for monitoring the lighting quality of the light emission of the luminaire 20.

The control system 10 may be a part of the luminaire 20. Such a luminaire 20 is an example of the luminaire according to the second aspect and the description of the luminaire of the second aspect is correspondingly valid. The control unit 2 of the control system 10 may be the control unit of the luminaire 20 configured to control operation of the luminaire 20.

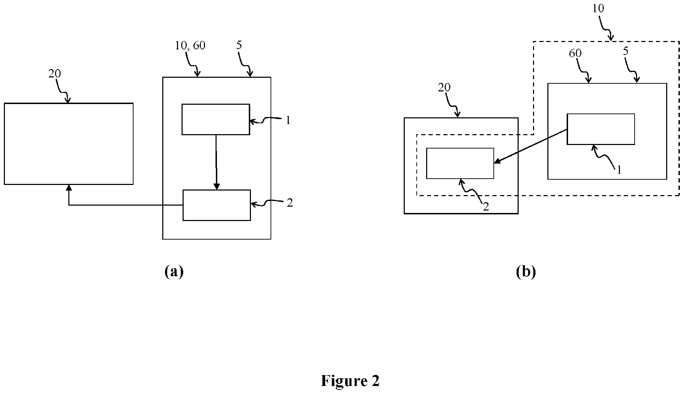

Further details on the control system, e.g. examples of implementation forms, are described in the following with regard to FIGs. 2, 5 and 6.

**FIG. 2** shows two examples of implementation forms of the control system of FIG. 1. According to the example of FIG. 2 (a), the control system 10 is a sensor device 60, wherein the sensor 1 and control unit 2 are arranged in a housing 5 of the sensor device 60. According to the example of FIG. 2 (b) the control system 10 comprises a sensor device 60 and the sensor 1 is arranged in a housing 5 of the sensor device 60. As shown in FIG. 2 (b), the control unit 2 of the control system may be a control unit of the luminaire 20.

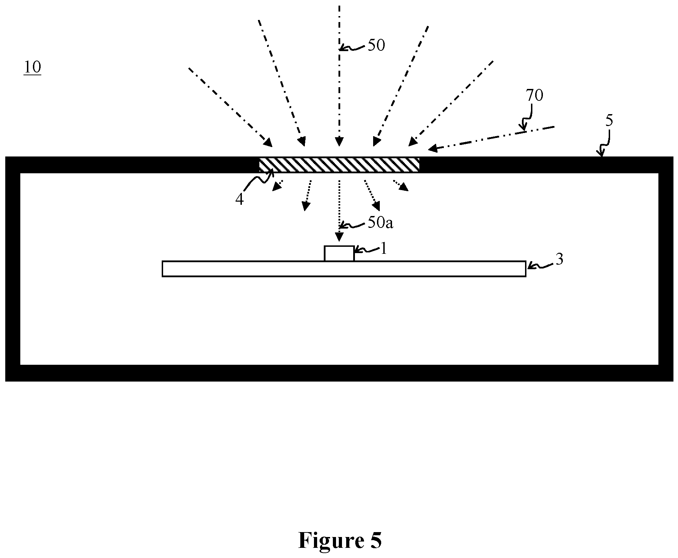

**FIG. 5** shows an example of an implementation form of the control system of FIG. 1. As shown in FIG. 5, the control system 10 may comprise a Lambertian diffusor 4 that is arranged such that the sensor 1 is configured to receive light only via the Lambertian diffusor 4. For achieving this, the sensor 1 may be arranged at a support structure 3, e.g. a printed circuit board, in a housing 5, wherein the housing 5 has an opening via which light may enter the housing 5 and reach the sensor 1 in order to be detected by the sensor 1. The opening is covered by the Lambertian diffusor 4 so that the light enters the housing 5 via said diffusor 4. As shown in FIG. 5, the light 50 reflected from the surface 30 being illuminated by the luminaire 20 travels through the diffusor 4. After having travelled through said diffusor a Lambertian distribution of the light is reached that is detected by the sensor 1. The Lambertian distribution of the light or the light filtered by the Lambertian diffusor 4 is indicated in Figure 5 by dotted arrows and labelled with the reference sign "50a". This light 50a is the light that enters the sensor 1. Incoming sunlight is shown with a dashed double dotted arrow in Figure 5 and labeled with the reference sign "70". Due to the sunlight having low angles of incident when reaching the diffusor 4, the amount of sunlight traveling through the diffusor 4 to the sensor is insignificant. That is, the light 50a entering the sensor 1 represents the light 50 reflected at the surface 30. Therefore, the implementation form of Figure 5 allows the sensor 1 to detect/measure the reflected light, wherein the detection results is insignificantly affected by sunlight. The housing 5 may be the housing of a sensor device or of the luminaire 20.

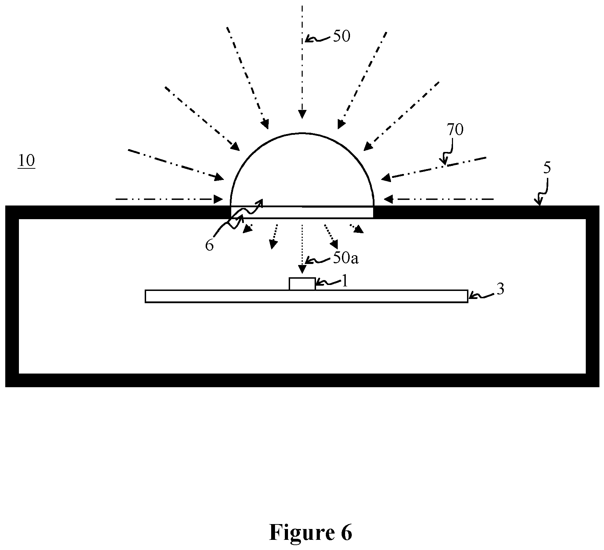

**FIG. 6** shows an example of an implementation form of the control system of FIG. 1. As shown in FIG. 6, the control system may comprise an optical dome structure 6 that is arranged such that the sensor 1 is configured to receive light only via the optical dome structure 6. For achieving this, the sensor 1 may be arranged at a support structure 3, e.g. a printed circuit board, in a housing 5, wherein the housing 5 has an opening via which light may enter the housing 5 and reach the sensor 1 in order to be detected by the sensor 1. The opening is covered by the optical dome structure 6 so that the light enters the housing 5 via said optical dome structure 6. As shown in FIG. 6, in contrast to the example of FIG. 5, the optical dome structure 6 allows the light 50 reflected from the surface 30 being illuminated by the luminaire 20 and incoming sunlight 70 to pass through and, thus, to reach the sensor 1 in the housing 1. Namely, the dome like structure 6 of the optical dome structure collects the rays coming from all directions as shown in Figure 6. The optical dome structure 6 may be realized with a volume scattering material or using a clear optic with an engrained surface structure. Thus, the light 50a entering the sensor 1 comprises the light 50 reflected at the surface and the sunlight 70. Therefore, the implementation form of Figure 6 allows the sensor 1 to detect/measure the reflected light and the sunlight. Optionally, the control system may comprise the sensor arrangement of Figure 6 (i.e. the sensor and the optical dome structure, irrespective how they are arranged) as a further sensor for detecting the artificial light (e.g. the light reflected at the surface 30) and the natural light (i.e. the sunlight) in addition to the sensor 1 of the control system 10. This may be the case when the Lambertian diffusor 4 is used for filtering the light entering the sensor 1.

Since the control system of the present invention allows determining the MEDI and optionally other lighting parameters, such as photopic illuminance, CCT etc., of the light emission of the luminaire and controlling the light emission of the luminaire according to the determined MEDI and optionally the other lighting parameters, the location of the luminaire e.g. being a free-standing luminaire, may be changed (e.g. to a room with a different ceiling), while ensuring that the light emission affects the circadian rhythm. The control of the light emission by the control unit of the control system allows adapting the spectrum and photopic illuminance of the luminaire to be efficient in melanopic activation.

In the claims as well as in the description the word "comprising" does not exclude other elements or steps and the indefinite article "a" or "an" does not exclude a plurality. A single element or other unit may fulfill the functions of several entities or items recited in the claims. The mere fact that certain measures are recited in the mutual different dependent claims does not indicate that a combination of these measures cannot be used in an advantageous implementation.

## Claims

1. A control system (10) for controlling light emission of a luminaire (20) towards a surface (30), the luminaire (20) being configured to emit light with wavelengths in the melanopic region, wherein the control system (10) comprises
- a sensor (1) configured to detect light and having a sensitivity in the melanopic region, and
- a control unit (2); wherein
- the sensor (1) is configured to be arranged such that the sensor (1) detects light (50) reflected from the surface (30) being illuminated by the luminaire when the luminaire emits light (40), and
- the control unit (2) is configured to
- determine a melanopic equivalent daylight illuminance, MEDI, of the light emission (40) of the luminaire (20) using a detection result of the sensor (1), and
- control the light emission (40) of the luminaire (20) according to the determined MEDI.

2. The control system (10) according to claim 1, wherein the control unit (2) is configured to control the luminaire (20) to emit light (40) with a spectral power distribution of
- a correlated color temperature, CCT, of 6500 Kelvin and a melanopic daylight efficacy ratio, MDER, equal to or greater than 1.15 in case the determined MEDI is greater than 0 lx and smaller than or equal to 250 lx;
- a CCT of 4000 Kelvin and a MDER equal to or greater than 0.9 and smaller than 1.15 in case the determined MEDI is greater than 250 lx and smaller than or equal to 500 lx; and
- a CCT of 4000 Kelvin and a MDER equal to or greater than 0.6 and smaller than 0.9 in case the determined MEDI is greater than 500 lx and smaller than or equal to 750 lx.

3. The control system (10) according to claim 2, wherein
- the control unit (2) is configured to control the luminaire (20) to emit no light in case the determined MEDI is greater than 750 lx.

4. The control system (10) according to any one of the previous claims, wherein
- the sensor (1) comprises a single sensor channel having a spectral sensitivity with a peak between 460 nm and 500 nm, optionally 475 nm and 485 nm.

5. The control system (10) according to any one of the previous claims, wherein
- the control unit (2) is configured to compute the MEDI by multiplying the detection result of the sensor (1) with a factor.

6. The control system (10) according to any one of claims 1 to 3, wherein
- the sensor (1) comprises multiple sensor channels having different spectral sensitivities, wherein a sensor channel of the multiple sensor channels has a spectral sensitivity with a peak between 460 nm and 500 nm, optionally 475 nm and 485 nm.

7. The control system (10) according to claim 6, wherein
- the control unit (2) is configured to compute the MEDI by summing up a multiplication of a detection result of each sensor channel of the multiple sensor channels with a respective factor.

8. The control system (10) according to any one of the previous claims, wherein
- the control system (10) comprises a Lambertian diffusor (4) that is arranged such that the sensor (1) is configured to receive light (50a) only via the Lambertian diffusor (4).

9. The control system (10) according to any one of claim 1 to 7, wherein
- the control system (10) comprises an optical dome structure (6) that is arranged such that the sensor (1) is configured to receive light (50a) only via the optical dome structure (6).

10. The control system (10) according to claim 9, when depending on claim 6, wherein
- an infrared channel of the multiple sensor channels is configured to detect infrared radiation, and
- the control unit (2) is configured to determine sunlight (70) detected by the sensor (1) using a detection result of the infrared channel of the multiple sensor channels and ignore the determined sunlight (70) for computing the MEDI.

11. The control system (10) according to any one of the previous claims, wherein the control unit (2) is configured to
- determine a photopic illuminance of light (50a) entering the sensor (1) using the detection result of the sensor (1), and
- control the light emission (40) of the luminaire (20) such that the greater the determined photopic illuminance of the light (50a) entering the sensor (2) the more the light emission (40) of the luminaire (20) is dimmed and vice versa.

12. The control system (10) according to any one of the previous claims, wherein
- the control unit (2) is configured to determine a spectral reflectivity of the surface (30) by computing a quotient of light (50a) detected by the sensor (1) and light (40) emitted by the luminaire (20).

13. The control system (10) according to any one of the previous claims, wherein
- the control unit (2) is configured to determine a total reflectivity of the surface (30) by computing a quotient of light detected by the sensor (1) and light emitted by the luminaire (20).

14. The control system (10) according to any one of the previous claims, wherein
- the control system (10) is a sensor device (60), the sensor (1) and control unit (2) being arranged in a housing (5) of the sensor device (60), or
- the control system (10) comprises a sensor device (60), the sensor (2) being arranged in a housing (5) of the sensor device (60).

15. A luminaire (20) for emitting light (40) towards a surface (30) comprising
- the control system (10) according to any one of the previous claims, wherein
- the luminaire (20) is configured to emit light with wavelengths in the melanopic region, and
- the sensor (1) of the control system (10) is arranged at the luminaire (20) such that the sensor (1) detects light (50) reflected from the surface (30) being illuminated by the luminaire (20) when the luminaire (20) emits light (40).

16. The luminaire (20) according to claim 15, wherein
- the control unit (2) of the control system (10) is a control unit of the luminaire (20) configured to control operation of the luminaire (20).
